# EUROPÄISCHE PATENTSCHRIFT

(11) **EP 2 648 659 B1**
(45) Veröffentlichungstag und Bekanntmachung des Hinweises auf die Patenterteilung: **25.02.2015**
(21) Anmeldenummer: 11788876.8
(22) Anmeldetag: 01.12.2011
(51) Int. Cl.: A61F 5/01, A61F 13/06

(54) **FUßGEWÖLBE-STÜTZSTRUMPF**
FOOT ARCH SUPPORT STOCKING
BAS DE CONTENTION POUR VOÛTE PLANTAIRE

(30) Priorität: 07.12.2010 DE 202010013071 U; 26.10.2011 DE 202011051761 U
(43) Veröffentlichungstag der Anmeldung: 16.10.2013
(73) Patentinhaber: Wendling, Dietrich, 70173 Stuttgart (DE)
(72) Erfinder: Wendling, Dietrich, 70173 Stuttgart (DE)
(74) Vertreter: Mammel und Maser
(86) Internationale Anmeldenummer: PCT/EP2011/071538
(87) Internationale Veröffentlichungsnummer: WO 2012/076398

(56) Entgegenhaltungen:
- WO-A1-2010/145818
- FR-A1- 2 881 641

## Beschreibung

Die Erfindung betrifft einen Fußgewölbe-Stützstrumpf, der aus einem Gewebematerial besteht.

Die derzeit zumeist am Markt erhältlichen Schuhe oder Schuhwerke unterstützen während eines Gangzyklusses den natürlichen Abrollvorgang eines Fußes nicht oder nur unzulänglich. Während eines Gangzyklusses verkürzt und verlängert sich der Fuß. Der Vorfuß und die Ferse drehen sich dabei gegeneinander, das heißt, in der Fußwurzel findet eine schraubenförmige Torsion und Gegentorsion statt, die zu einer Längenänderung des Fußes und einer Höhenänderung des Fußgewölbes führen. In einer Abstoßphase hebt sich das Fußgewölbe und der Fuß verkürzt sich, in der vorangegangenen Standphase senkt er sich, und der Fuß wird dadurch länger. Zwischen den gegensätzlichen Bewegungen besteht eine physiologische Balance, welche beim Senk-Spreiz-Fuß zugunsten der Streckung und Abflachung verschoben ist. Der Fuß wird dadurch insgesamt länger, breiter und flacher. Da die Sehnen und Muskeln der Ausdehnung des Fußes häufig nicht folgen, werden Zehendeformationen begünstigt, wie beispielsweise Krallen- und Hammerzehen oder Hallux valgus.

Aus der FR 2 881 641 A1 ist ein Fußgewölbe-Stützstrumpf bekannt, der aus einem Gewebematerial besteht, welches ein Vorfußabschnitt, ein Mittelfußabschnitt und einen Rückfußabschnitt mit zumindest einem Fersenbereich aufweist.

Der Erfindung liegt deshalb die Aufgabe zugrunde, ein Gleichgewicht zwischen Torsion und Gegentorsion, zwischen Anhebung und Absenkung des Fußgewölbes sowie zwischen Fuß-Kontraktion und Fuß-Ausdehnung zugunsten der Anhebung des Gewölbes und der Fuß-Kontraktion zu schaffen.

Diese Aufgabe wird erfindungsgemäß durch einen Fußgewölbe-Stützstrumpf gelöst, der aus einem Gewebematerial besteht, welches einen Vorfußabschnitt, einen Mittelfußabschnitt im Bereich eines Fußgewölbes und ein Rückfußabschnitt sowie zumindest einen Fersenbereich aufweist, der die Ferse umgreift, wobei zumindest ein elastischer Zug vorgesehen ist, der sich ausgehend von einer Außenseite des Rückfußabschnittes entlang einer Fußsohle diagonal verlaufend bis zur Innenseite des Mittel- oder Vorfußabschnittes erstreckt oder sich weiter über einen Rist bis zur Außenseite des Mittel- oder Vorfußabschnittes erstreckt. Die erste Alternative weist den Vorteil auf, dass durch diesen diagonal verlaufenden elastischen Zug entlang der Fußsohle eine zusätzliche Kontraktionskraft zwischen dem Vorfuß und dem Rückfuß aufgebracht wird, um die Bildung des Fußgewölbes oder die Aufrichtung des Fußgewölbes zu unterstützen. Dadurch wird eine bogenartige Vorspannung des Fußgewölbes ermöglicht, welche während eines Gangzyklusses einem Absenken und einer Streckung des Fußes und somit auch den daraus folgenden Zehendeformationen entgegenwirkt. Dadurch kann eine helikale Verdrehung zwischen dem Vorfuß und dem Rückfuß erfolgen. Es wird also durch diesen Fußgewölbe-Stützstrumpf die Wiederherstellung des Fußgewölbes während eines Gangzyklusses durch den zumindest einen elastischen Zug gefördert und die Kontraktion und Torsion des Fußes unterstützt. Dieser zumindest eine elastische Zug bewirkt einen sogenannten Push-Off-Support. Insbesondere durch den entlang unter der Fußsohle sich hindurch erstreckenden ersten elastischen Zug wird eine Kraftunterstützung in etwa dem Verlauf der Sehne des M. peronaeus longus erzielt. Zusätzlich unterstützt dieser elastische Zug, der bevorzugt im Vorfußbereich beginnt, neben dem M. fibularis longus auch noch die Zehenbeuger sowie die Abspreizer der Großzehe im Sinne eine Hallux-valgus-Korrektur. Durch die weitere Alternative, dass dieser erste elastische Zug sich weiter über die Innenseite entlang des Mittel- oder Vorfußes bis zu dessen Außenseite erstreckt, werden die vorgenannte Wirkung und die Vorteile verstärkt, insbesondere die Torsion des Fußes durch Einwärtsdrehung der Ferse und Abwärtsdrehung der Innenseite des Vorfußes.

Nach einer weiteren bevorzugten Ausgestaltung der Erfindung ist ein weiterer beziehungsweise zweiter elastischer Zug vorgesehen, der sich von einer Innenseite des Rückfußabschnittes über den Rist zur Außenseite bis zum Mittel- oder Vorfußabschnitt erstreckt. Dieser weitere beziehungsweise zweite elastische Zug unterstützt des Weiteren die Torsion zwischen Rückfuß und Vorfuß.

Des Weiteren ist bevorzugt vorgesehen, dass der weitere beziehungsweise zweite elastische Zug über den Rist nach distal lateral bis in die Gegend des fünften Mittelfußknochens beziehungsweise der Kleinzehe sich erstreckt. Bei dieser Ausgestaltung unterstützt dieser elastische Zug insbesondere den M. tibialis posterior. Dadurch kann eine weitere Abrollbewegung und Wiederherstellung des Fußgewölbes unterstützt werden.

Eine weitere bevorzugte Ausgestaltung des Fußgewölbe-Stützstrumpfes sieht vor, dass der zweite elastische Zug sich weiter von der Außenseite des Mittelfußabschnittes oder Vorderfußabschnittes entlang der Fußsohle bis zur Innenseite des Mittel- oder Vorfußabschnitts erstreckt. Dadurch kann nach einer Aufspreizung des Vorfußes eine Rückkontraktion erzielt werden. Bevorzugt ist vorgesehen, dass dieser zweite elastische Zug von der Innenseite des Mittel- oder Vorfußabschnittes noch weiter bis zum Rist oder bis zur Außenseite über den Rist zum Mittel- oder Vorfußabschnitt geführt ist und insbesondere eine volle Umschlingung des Mittel- oder Vorfußabschnittes ermöglicht.

Eine weitere alternative Ausgestaltung des Fußgewölbestützstrumpfes sieht am Ende des zweiten elastischen Zuges, der sich über den Rist nach distal-lateral bis in die Gegend des fünften Mittelfußknochens beziehungsweise der Kleinzehe erstreckt, einen dritten elastischen Zug vor, der sich von der Außenseite des Mittelfußabschnittes oder des Vorfußabschnittes entlang der Fußsohle bis zur Innenseite des Mittel- oder Vorfußabschnittes erstreckt beziehungsweise am Ende des zweiten elastischen Zuges angreift und sich dann von der Außenseite des Mitteloder Vorfußabschnittes entlang der Fußsohle bis zur Innenseite des Mitteloder Vorfußabschnitts erstreckt. Dieser dritte elastische Zug kann sich in einer dritten alternativen Ausführungsform auch noch weiter bis zum Rist oder sogar bis zur Außenseite des Mittel- oder Vorfußabschnittes erstrecken und somit wieder zurück geführt werden, so dass dieser eine volle Umschlingung aufweist. Dadurch kann dieser weitere dritte elastische Zug im Sinne einer Vorfuß- oder Mittelfußbandage ausgebildet sein.

Das freie Ende des vorzugsweise dritten elastischen Zuges ist nach dem Umschlingen des Vor- oder Mittelfußabschnittes bevorzugt an dem diagonal über den Rist verlaufenden Abschnitt des zweiten elastischen Zuges fixiert. Dadurch kann insbesondere ermöglicht werden, dass der zweite Zug in den dritten Zug übergeht und das Ende des dritten Zuges wieder mit dem zweiten Zug im Bereich des über den Rist verlaufenden diagonalen Abschnittes verbunden ist. Somit kann eine Art Rundtour oder Umschlingung um den Vorfuß- oder Mittelfußabschnitt ermöglicht werden, wobei die Züge, vorzugsweise lösbar, miteinander verbunden sind. Gleiches gilt, wenn der dritte elastische Zug eine Verlängerung des zweiten Zuges darstellt, so dass der dritte Zug ein Teil des zweiten Zugs ist.

Eine weitere bevorzugte Ausgestaltung sieht vor, dass ein Ende des ersten elastischen Zuges am Rückfußabschnitt und ein Ende des zweiten elastischen Zuges am Rückfußabschnitt, insbesondere im Fersenbereich, miteinander verbunden sind. Dadurch kann eine optimale Unterstützung für die Wiederherstellung des Fußgewölbes nach dessen Belastung beziehungsweise Streckung gegeben sein.

In dem Gewebematerial ist der zumindest eine elastische Zug bevorzugt eingearbeitet, so dass dieser quasi in dem Gewebematerial integriert ist. Alternativ kann der zumindest eine elastische Zug auf einer Innen- oder Außenseite des Gewebematerials aufgebracht sein.

Eine alternative Ausführungsform besteht darin, dass der zumindest eine elastische Zug mit einem Ende fest mit dem Gewebematerial verbunden und das andere Ende lösbar zum Gewebematerial vorgesehen ist. Diese lösbare Verbindung kann beispielsweise durch einen Klettverschluss, einen Einhängeverschluss oder dergleichen ermöglicht sein. Diese Ausführungsform weist den Vorteil auf, dass ein leichteres Anziehen des Fußgewölbe-Stützstrumpfes gegeben ist, da erst nach dem Überstülpen des Gewebematerials über den Fuß die Vorspannung des zumindest einen elastischen Zuges aufgebracht wird. Zusätzlich kann bei dieser Ausführungsform individuell die Spannkraft oder die Kraft zur Wiederherstellung des Fußgewölbes eingestellt werden.

Eine weitere bevorzugte Ausgestaltung sieht vor, dass der zumindest eine elastische Zug mit beiden Enden lösbar an dem Gewebematerial angreift. Dadurch kann ein flexibler Fußgewölbe-Stützstrumpf bereitgestellt werden, der bezüglich der Spannkraft der Züge individuell auf die Belastungsfälle einstellbar ist.

Der zumindest eine zum Einsatz kommende Zug des Fußgewölbe-Stützstrumpfes ist als Kraftspeicherelement, insbesondere aus gummielastischem Material oder als gummielastisches Band, ausgebildet.

Die Erfindung sowie weitere vorteilhafte Ausführungsformen und Weiterbildungen derselben werden im Folgenden anhand der in den Zeichnungen dargestellten Beispiele näher beschrieben und erläutert. Die der Beschreibung und den Zeichnungen zu entnehmenden Merkmale können einzeln für sich oder zu mehreren in beliebiger Kombination erfindungsgemäß angewandt werden. Es zeigen:
Figur 1 a eine schematische Ansicht von oben auf eine Knochenstruktur des Fußes,
Figur 1b eine schematische Seitenansicht auf die Knochenstruktur des Fußes,
Figuren 2a und b eine erste Ausführungsform des Fußgewölbe-Stützstrumpfes,
Figur 2c eine alternative Ausführungsform zu den Figuren 2a und 2b,
Figuren 3a und b eine weitere alternative Ausführungsform des Fußgewölbe-Stützstrumpfes zu den Figuren 2,
Figur 4 eine weitere schematische Ansicht der Ausführungsform gemäß Figur 3 und
Figur 5 eine weitere alternative Ausgestaltung des Fußgewölbe-Stützstrumpfes zu Figur 2.

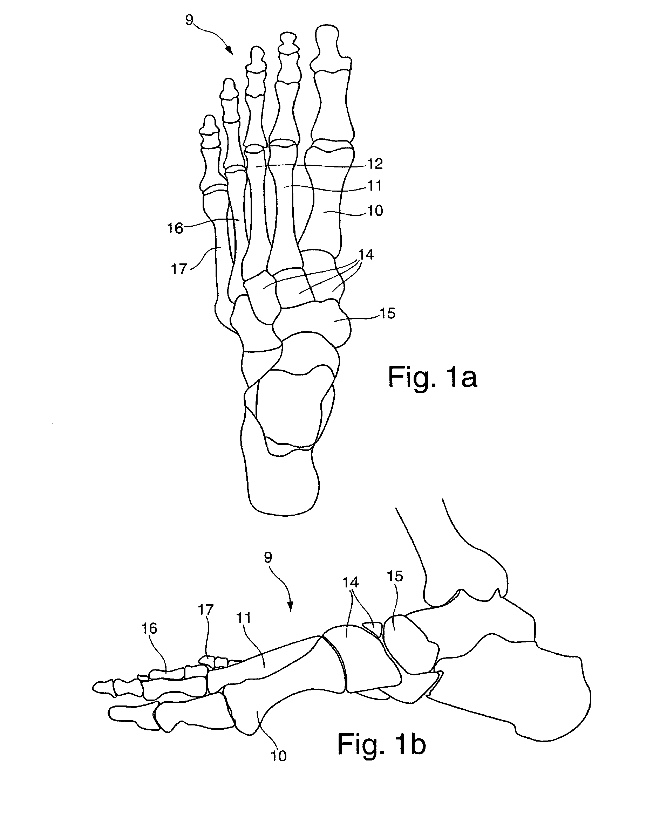

In den Figuren 1 a und 1 b ist eine Knochenstruktur des Fußes 9 dargestellt, dessen Aufbau allgemein bekannt ist. Für die Beschreibung der nachfolgenden Erfindung wird lediglich dargestellt, was unter dem ersten bis fünften Strahl 10, 11, 12, 16, 17 zu verstehen ist. Der erste, zweite und dritte Strahl 10, 11, 12 des Fußes betreffen die Mittelfußknochen, welche über jeweils ein Keilbein 14 mit einem Kahnbein 15 in Verbindung stehen. Dieser erste bis dritte Strahl 10, 11, 12 bewegt sich relativ zum vierten und fünften Strahl 16, 17, das heißt, dass der erste bis dritte Strahl 10, 11, 12 an der Abflachung und Anhebung des Fußgewölbes sowie einer Verlängerung und Verkürzung des Fußes 9 unmittelbar nach dem Abrollen der Ferse beteiligt sind. Der vierte und fünfte Strahl 16, 17 bilden die Torsionsachse bei Abrollung des Vorfußes. Da sie weniger mobil sind als die medialen Strahlen und bei Belastung weniger nachgeben, lenken diese die Kraft und das Gewicht am Ende der Abrollphase auf die Großzehe.

Der natürliche Abrollvorgang eines Fußes lässt sich in vier Phasen unterscheiden:
1. Beim Aufsetzen der Ferse wird die Energie des Aufpralls teilweise über den Sprungbeinkopf in das Längsgewölbe geführt, indem dieses angespannt wird.
2. Diese Spannung verstärkt sich weiter, indem das Körpergewicht auf den Gewölbebogen einwirkt. Hierbei senkt sich das Längs- und Quergewölbe. Es kommt zu einer Abflachung des Fußgewölbes, was gleichzeitig mit einer Verlängerung des Fußes einhergeht.
3. Die elastische Spannung erreicht ihr Maximum beim Abstoßen vom Boden,
4. sodann geht die Spannungsenergie wieder in Bewegungsenergie über, was dem Körper einen Impuls nach vorne erlaubt. Die Großzehenbewegung sorgt dabei für eine möglichst gute Bodenhaftung und für einen letzten Schub vor dem Anheben des Beines. Während dieser Phase hebt sich das Fußgewölbe wieder an, und es kommt zu einer Verkürzung des Gesamtfußes.

Wenn das Fußlängsgewölbe sich anhebt und verkürzt oder sich senkt oder verlängert, geschieht dies vor allem auf der medialen (inneren) Seite des Fußes während die laterale (äußere) Seite davon weitgehend unbeeinflusst bleibt. So entsteht im Mittelfuß-Fußwurzelgelenk eine Abduktions- und Adduktionsbewegung. Der erfindungsgemäße Fußgewölbe-Stützstrumpf unterstützt diese Bewegung und den natürlichen Abrollvorgang des Fußes.

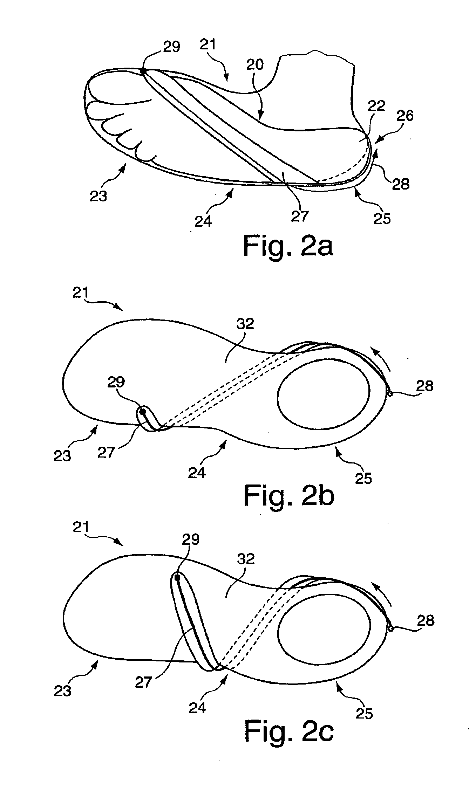

In Figur 2a ist eine erste schematische Ansicht einer ersten erfindungsgemäßen Ausführungsform eines Fußgewölbe-Stützstrumpfes 21, und in Figur 2b ist eine weitere Ansicht von oben auf die Ausführungsform in Figur 2a dargestellt. Dieser Fußgewölbe-Stützstrumpf 21 besteht aus einem Gewebematerial 22, welches zumindest einen Vorfußabschnitt 23, einen Mittelfußabschnitt 24 und einen Rückfußabschnitt 25 umfasst, der zumindest einen Fersenbereich 26 umgreift. Bei diesem Gewebematerial 22 kann es sich um ein Gewebe aus natürlichen oder künstlichen Fasern handeln, welches aufgrund deren Gewebestruktur eine gewisse Nachgiebigkeit aufweist, jedoch eine zumindest geringe Stützwirkung auf den gesamten Fuß ausübt.

Die erste Ausführungsform des Fußgewölbe-Stützstrumpfes 21 umfasst einen ersten elastischen Zug 27, der als Kraftspeicherelement, wie beispielsweise ein Gummiband, ausgebildet ist und beispielsweise in das Gewebematerial eingearbeitet oder auf einer Innen- oder Außenseite aufgebracht, auflaminiert, aufkaschiert oder dergleichen ist. Der elastische Zug 27 kann, wie dies in Figur 2 dargestellt ist, als Band ausgebildet sein, welches in der Breite vorzugsweise an die Körpergröße und/oder das Körpergewicht angepasst ist. Die innerhalb des Bandes dargestellte Linie stellt die Wirkrichtung und den Verlauf der Kraftrichtung dar. Der elastische Zug 27 kann anstelle eines Bandes auch auf einen dünnen linienförmigen Zug reduziert sein. Der erste elastische Zug 27 beginnt mit einem Ende 28 an einer Außenseite des Rückfußabschnittes 25 oder im Fersenbereich 26 und verläuft dann entlang einer Fußsohle diagonal zur Innenseite des Mittelfußabschnittes 24, insbesondere des Vorfußabschnittes 25, und endet vorzugsweise seitlich oberhalb der Fußsohle mit seinem zweiten Ende 29. Dieser Verlauf entspricht in etwa dem Verlauf der Sehne des M. peronaeus longus und unterstützt diese Sehne bei der Wiederherstellung des Fußgewölbes 20 nach der Belastung des Fußes. Alternativ kann das zweite Ende 29, wie in Figur 2b dargestellt ist, in Bereichen des vierten oder fünften Strahles 16, 17 enden, wie dies in Figur 2c dargestellt ist.

Dieser erste elastische Zug 27 kann beispielsweise im Rückfußabschnitt 25 fest mit dem Gewebematerial 22 verbunden sein und am gegenüberliegenden Ende 29 lösbar, beispielsweise mit einem Klettverschluss, mit dem Gewebematerial 22 verbindbar sein, so dass eine entsprechende Vorspannung des ersten elastischen Zuges 27 einstellbar ist. Ebenso können das feste und lose Ende 28, 29 auch vertauscht angeordnet sein. Alternativ können auch beide Enden 28, 29 des ersten elastischen Zuges 27 lösbar sein, so dass nach dem Überziehen des Fußgewölbe-Stützstrumpfes der erste elastische Zug 27 befestigbar ist. Zur besseren Lagefixierung des ersten elastischen Zuges 27 kann entlang des vorgesehenen Verlaufs auf dem Gewebematerial eine Gummierung aufgebracht sein, so dass nach dem Anlegen des ersten elastischen Zuges 27 eine bessere Haftung und Fixierung gegeben ist.

Der erste elastische Zug 27 kann sich von einer Außenseite des Rückfußabschnittes 25 noch bis zu einem hinteren Bereich der Ferse erstrecken, so dass der Rückfußabschnitt 25 im Kraftwirkungsbereich des ersten elastischen Zuges mit eingebunden ist.

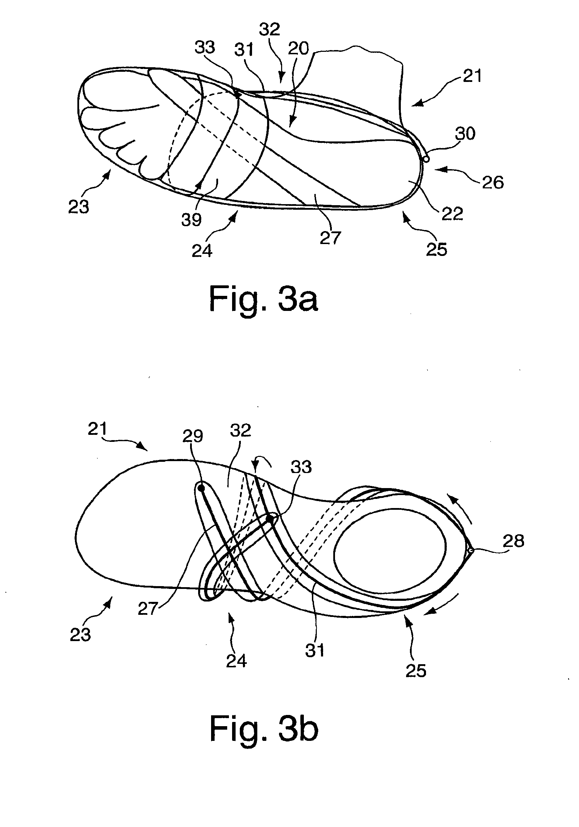

Ergänzend können in dem Gewebematerial Zonen 39, wie dies in Figur 3a dargestellt ist, eingearbeitet sein, welche eine höhere Kompressionswirkung als die benachbarten Bereiche aufweisen.

In Figur 3a und Figur 3b ist eine weitere alternative Ausführungsform der Erfindung vorgesehen. Ergänzend zum ersten elastischen Zug 27 ist bevorzugt ein weiterer Zug oder zweiter Zug 31 vorgesehen. Dieser zweite elastische Zug 31 erstreckt sich von einem Rückfußabschnitt 25 entlang der Innenseite des Fußes, vorzugsweise unterhalb eines Knöchels, über den Rist 32 bis zum äußeren Bereich des Mittelfußabschnitts 24, vorzugsweise Vorfußabschnitt 23. Insbesondere erstreckt sich dieser zweite elastische Zug 31 bis in die Gegend des fünften Mittelfußknochens beziehungsweise der Kleinzehe. Dadurch kann der M. tibialis posterior unterstützt werden. Vorteilhafterweise kann vorgesehen sein, dass sich der zweite elastische Zug 31 vom Bereich des fünften Mittelfußknochens beziehungsweise der Kleinzehe weiter entlang der Fußsohle bis zum Großballen erstreckt oder sich insbesondere bis nach oben erstreckt, so dass das zweite Ende 33 des zweiten elastischen Zuges 31 eine Umschlingung des Vorfußabschnittes 23 oder eine Art Mittelfußbandage bildet. Die Anbringungs- und Befestigungsarten und Lösungsmöglichkeiten des zweiten elastischen Zuges 31 entsprechen denen des ersten elastischen Zuges 27. Ergänzend kann beim zweiten elastischen Zug 31 vorgesehen sein, dass ein freies Ende 33 des zweiten Zuges 31 nach der Bildung einer Mittelfußbandage an einem Abschnitt des zweiten Zuges 31 endet oder vorzugsweise lösbar befestigbar ist, der diagonal über den Rist 32 geführt ist. Gemäß einer ersten Ausführungsform kann das zweite Ende 33 des zweiten elastischen Zuges 31 an einem Abschnitt des zweiten elastischen Zuges 31 angreifen, der im Bereich des Ristes 32 oder eher außerhalb des Ristes 32 liegt, so dass eine Umschlingung gebildet ist. Alternativ kann dieses zweite Ende 33 im Bereich des zweiten Endes 29 des ersten Zuges 27 liegen. Es kann aber auch vorgesehen sein, dass das zweite Ende 29 des ersten Zuges 27 von einem Abschnitt des zweiten Zuges 31 im Bereich des Ristes 32 oder an der Außenseite überdeckt ist und deckungsgleich liegt.

Durch die Kombination des ersten und zweiten Zuges 27, 31, wie dies in den Figuren 3a und b dargestellt ist, kann durch deren Verlauf und Anordnung eine verstärkte Aufstellung des Fußgewölbes und gleichzeitig eine helikale Verdrehung zwischen dem Mittel- und Vorfuß zum Rückfuß erzielt werden, wodurch der natürliche Abrollvorgang unterstützt und anschließend das Fußgewölbe wieder aufgestellt wird, ohne dass dabei eine Behinderung oder Beschränkung der Beweglichkeit des Fußes gegeben ist.

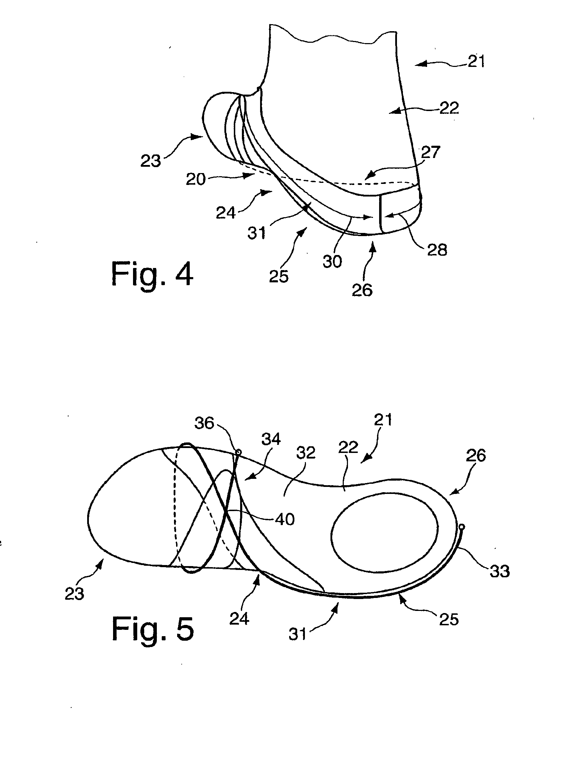

In Figur 4 ist eine weitere schematische Seitenansicht des Fußgewölbe-Stützstrumpfes 21 mit dem ersten und zweiten Zug 27, 31 dargestellt. Das erste Ende 28 des ersten elastischen Zuges 27 und das erste Ende des zweiten elastischen Zuges 31 können aneinander grenzen oder miteinander verbunden sein.

Die Figur 5 zeigt eine weitere alternative Ausführungsform zu Figur 3, wobei der Fußgewölbe-Stützstrumpf 21 gemäß Figur 5 gegenüber dem in Figur 3 dahingehend modifiziert ist, dass der zweite Zug 31 sich über die Schnittstelle oder den Kreuzungspunkt 40 auf dem Rist 32 hinweg erstreckt und wiederum an der Außenseite des Mittelfußabschnittes 24 endet. Alternativ zu dieser Ausführungsform kann der zweite elastische Zug 31 derart verlaufen, wie dies in Figur 3 beschrieben ist, und ein weiterer elastischer Zug 34 beziehungsweise dritter elastischer Zug 34 erstreckt sich seitlich von einer Innenseite im Bereich des Fußballens ausgehend über den Rist 32 bis zur Außenseite des Mittelfußabschnittes 24. Die Befestigungsmöglichkeiten und Alternativen dieses dritten elastischen Zuges 34 können dem des ersten elastischen Zuges 27 entsprechen.

Alternativ können bei diesem Fußgewölbe-Stützstrumpf 21 in dem Gewebematerial auch einzelne Laschen eingearbeitet oder gegeben sein, welche zur Führung der Züge 27, 31, 34 vorgesehen sind beziehungsweise in welche die Züge 27, 31, 34 eingefädelt werden können.

Die zu Figur 2 beschriebene Ausgestaltung des elastischen Zuges 27 gilt in Analogie für die weiteren Züge 31 und 34.

Dieser Fußgewölbe-Stützstrumpf 21 wird insbesondere wie eine herkömmliche Socke getragen, so dass die Benutzung eines solchen Fußgewölbe-Stützstrumpf 21 nicht sichtbar ist. Alternativ kann dieser Fußgewölbe-Stützstrumpf 21 eine verstärkte Laufsohle aufweisen, so dass diese als Hausschuhe, Turnschuhe oder dergleichen einsetzbar sind.

## Patentansprüche

1. Fußgewölbe-Stützstrumpf bestehend aus einem Gewebematerial (22), welches einen Vorfußabschnitt (23), einen Mittelfußabschnitt (24) und einen Rückfußabschnitt (25) mit zumindest einem Fersenbereich (26) aufweist, **dadurch gekennzeichnet, dass** zumindest ein erster elastischer Zug (27) vorgesehen ist, der sich ausgehend von einer Außenseite des Rückfußabschnittes (25) entlang einer Fußsohle diagonal verlaufend bis zur Innenseite des Mittel- oder Vorfußabschnittes (24, 23) erstreckt oder weiter über einen Rist (32) bis zur Außenseite des Mittel- oder Vorfußabschnitts (24, 23) erstreckt.

2. Fußgewölbe-Stützstrumpf nach Anspruch 1, **dadurch gekennzeichnet, dass** ein weiterer, zweiter elastischer Zug (31) vorgesehen ist, der sich von einer Innenseite des Rückfußabschnittes (25) ausgehend über einen Rist (32) bis zur Außenseite des Mittelfußabschnitts (24) oder Vorfußabschnitt (23) erstreckt.

3. Fußgewölbe-Stützstrumpf nach Anspruch 2, **dadurch gekennzeichnet, dass** sich der zweite elastische Zug (31) bis in den Bereich des fünften Mittelfußknochens oder der Kleinzehe erstreckt.

4. Fußgewölbe-Stützstrumpf nach einem der vorhergehenden Ansprüche, **dadurch gekennzeichnet, dass** sich der zweite elastische Zug (31) ausgehend von der Außenseite des Mittelfußabschnittes (24) oder des Vorfußabschnittes (23) weiter entlang der Fußsohle bis zur Innenseite des Mittel- oder Vorfußabschnitts (24, 23) erstreckt und vorzugsweise bis zum Rist (32) oder wieder zur Außenseite des Mittel- oder Rückfußabschnittes (24, 23) über den Rist (32) zurückgeführt ist und eine volle Umschlingung aufweist.

5. Fußgewölbe-Stützstrumpf nach einem der Ansprüche 1 bis 3, **dadurch gekennzeichnet, dass** ein dritter elastischer Zug (34) vorgesehen ist, der sich von der Außenseite des Mittelfußabschnittes (24) oder des Vorfußabschnittes (23) entlang der Fußsohle bis zur Innenseite des Mittel- oder Vorfußabschnittes (24, 23) erstreckt und vorzugsweise bis zum Rist (32) oder zur Außenseite des Mitteloder Rückfußabschnittes (24, 25) wieder zurückgeführt ist und vorzugsweise eine volle Umschlingung aufweist.

6. Fußgewölbe-Stützstrumpf nach einem der Ansprüche 1 bis 4, **dadurch gekennzeichnet, dass** ein Ende (33) des zweiten elastischen Zuges (31) nach dem Umschlingen des Vor- oder Mittelfußabschnittes (23, 24) mit dem diagonal über den Rist (32) verlaufenden Abschnitt des zweiten Zuges (31) lösbar verbunden oder daran fixiert ist.

7. Fußgewölbe-Stützstrumpf nach Anspruch 1 oder 2, **dadurch gekennzeichnet, dass** ein Ende (28) des ersten Zuges (27) am Rückfußabschnitt (25) und ein Ende (30) des zweiten Zuges (31) am Rückfußabschnitt (25) miteinander verbindbar sind oder im Fersenbereich (26) angeordnet oder miteinander verbindbar sind.

8. Fußgewölbe-Stützstrumpf nach einem der vorhergehenden Ansprüche, **dadurch gekennzeichnet, dass** zumindest ein elastischer Zug (27, 31, 34) in das Gewebematerial eingearbeitet oder auf einer Außen- oder Innenseite des Gewebematerials fest angeordnet vorgesehen ist.

9. Fußgewölbe-Stützstrumpf nach einem der vorhergehenden Ansprüche, **dadurch gekennzeichnet, dass** zumindest ein Ende (28, 29, 30, 36) des zumindest einen elastischen Zuges (27, 31, 34) fest mit dem Gewebematerial (22) verbunden und das andere Ende (29, 33, 35) als zumindest ein elastischer Zug (27, 31, 34) fest an dem Gewebematerial (22) befestigt ist.

10. Fußgewölbe-Stützstrumpf nach einem der vorhergehenden Ansprüche, **dadurch gekennzeichnet, dass** zumindest ein Ende (28, 29, 30, 33, 35, 36) des zumindest einen elastischen Zuges (34) lösbar mit dem Gewebematerial (22) verbindbar sind.

11. Fußgewölbe-Stützstrumpf nach einem der vorhergehenden Ansprüche, **dadurch gekennzeichnet, dass** zumindest ein lösbares Ende (28, 29, 30, 33, 35, 36) des zumindest einen Zuges (27, 31, 34) zur Einstellung einer Vorspannkraft lösbar an dem Gewebematerial (22) befestigt ist.

12. Fußgewölbe-Stützstrumpf nach einem der vorhergehenden Ansprüche, **dadurch gekennzeichnet, dass** der zumindest eine Zug (27, 31, 34) als ein Kraftspeicherelement, insbesondere als ein gummielastisches Band, ausgebildet ist.

## Claims

1. A foot arch support stocking consisting of a fabric material (22) which has a forefoot portion (23), a midfoot portion (24) and a hind foot portion (25) including at least a heel region (26), **characterised in that** at least one first elastic traction element (27) is provided which, beginning from an outer side of the hind foot portion (25) and passing diagonally along a foot sole, extends to the inner side of the midfoot portion or forefoot portion (24, 23) or extends farther across an instep (32) to the outer side of the midfoot portion or forefoot portion (24, 23).

2. The foot arch support stocking as claimed in claim 1, **characterised in that** another, second elastic traction element (31) is provided which, beginning from an inner side of the hind foot portion (25), extends across an instep (32) to the outer side of the midfoot portion (24) or forefoot portion (23).

3. The foot arch support stocking as claimed in claim 2, **characterised in that** the second elastic traction element (31) extends to the region of the fifth metatarsal or of the little toe.

4. The foot arch support stocking as claimed in any of the preceding claims, **characterised in that** the second elastic traction element (31), beginning from the outer side of the midfoot portion (24) or of the forefoot portion (23), extends farther along the foot sole to the inner side of the midfoot portion or the forefoot portion (24, 23) and is preferably passed on to the instep (32) or is passed on farther across the instep (32) and back again to the outer side of the midfoot portion or hind foot portion (24, 23), thus forming a full wrap.

5. The foot arch support stocking as claimed in any one of claims 1 to 3, **characterised in that** a third elastic traction element (34) is provided, which, beginning from the outer side of the midfoot portion (24) or of the forefoot portion (23), extends along the foot sole to the inner side of the midfoot portion or of the forefoot portion (24, 23) and is preferably passed on to the instep (32) or is passed on back again to the outer side of the midfoot portion or hind foot portion (24, 25), preferably forming a full wrap.

6. The foot arch support stocking as claimed in any one of claims 1 to 4, **characterised in that** one end (33) of the second elastic traction element (31), once wrapped around the forefoot portion or midfoot portion (23, 24), is releasably connected, or secured, to the portion of the second traction element (31) extending diagonally across the instep (32).

7. The foot arch support stocking as claimed in claim 1 or 2, **characterised in that** an end (28) of the first traction element (27) disposed in the hind foot portion (25) and an end (30) of the second traction element (31) disposed in the hind foot portion (25) are connectable to each other, or are disposed, or connectible to each other, in the heel region (26).

8. The foot arch support stocking as claimed in any of the preceding claims, **characterised in that** at least one elastic traction element (27, 31, 34) is provided and incorporated into the fabric material or firmly arranged on an outer side or on an inner side of the fabric material.

9. The foot arch support stocking as claimed in any of the preceding claims, **characterised in that** at least one end (28, 29, 30, 36) of the at least one elastic traction element (27, 31, 34) is firmly connected with the fabric material (22) and the other end (29, 33, 35) is firmly fastened to the fabric material (22) as at least one elastic traction element (27, 31, 34).

10. The foot arch support stocking as claimed in any of the preceding claims, **characterised in that** at least one end (28, 29, 30, 33, 35, 36) of the at least one elastic traction element (34) is releasably connectable with the fabric material (22).

11. The foot arch support stocking as claimed in any of the preceding claims, **characterised in that** at least one releasable end (28, 29, 30, 33, 35, 36) of the at least one traction element (27, 31, 34) is releasably fastened to the fabric material (22) so as to allow the setting of a preload force.

12. The foot arch support stocking as claimed in any of the preceding claims, **characterised in that** the at least one traction element (27, 31, 34) is realised in the form of an energy storing member, in particular a rubber-elastic band.

## Revendications

1. Bas de contention pour voûte plantaire composé d'un matériau textile (22) qui présente une partie avant-pied (23), une partie mi-pied (24) et une partie arrière-pied (25) avec au moins une zone talon (26), **caractérisé en ce qu'**il est prévu au moins un premier élément de traction élastique (27) qui s'étend, à partir d'une face extérieure de la partie arrière-pied (25), en diagonal le long d'une plante de pied jusqu'à la face intérieure de la partie mi-pied ou avant-pied (24, 23) ou, au-delà, jusqu'à la face extérieure de la partie mi-pied ou avant-pied (24, 23) en passant par un coup de pied (32).

2. Bas de contention pour voûte plantaire selon la revendication 1, **caractérisé en ce qu'**il est prévu un autre, deuxième élément de traction élastique (31) qui s'étend, à partir d'une face intérieure de la partie arrière-pied (25), jusqu'à la face extérieure de la partie mi-pied (24) ou de la partie avant-pied (23) en passant par un coup de pied (32).

3. Bas de contention pour voûte plantaire selon la revendication 2, **caractérisé en ce que** le deuxième élément de traction élastique (31) s'étend jusque dans la zone du cinquième métatarse ou du petit orteil.

4. Bas de contention pour voûte plantaire selon l'une quelconque des revendications précédentes, **caractérisé en ce que** le deuxième élément de traction élastique (31) s'étend, à partir de la face extérieure de la partie mi-pied (24) ou de la partie avant-pied (23), plus loin le long de la plante de pied jusqu'à la face intérieure de la partie mi-pied ou avant-pied (24, 23) et revient de préférence jusqu'au coup de pied (32) ou jusqu'à la face extérieure de la partie mi-pied ou arrière-pied (24, 23) en passant par le coup de pied (32), et présente un tour complet.

5. Bas de contention pour voûte plantaire selon l'une quelconque des revendications 1 à 3, **caractérisé en ce qu'**il est prévu un troisième élément de traction élastique (34) qui s'étend, à partir de la face extérieure de la partie mi-pied (24) ou de la partie avant-pied (23), le long de la plante de pied jusqu'à la face intérieure de la partie mi-pied ou avant-pied (24, 23) et revient de préférence jusqu'au coup de pied (32) ou jusqu'à la face extérieure de la partie mi-pied ou arrière-pied (24, 25) et présente de préférence un tour complet.

6. Bas de contention pour voûte plantaire selon l'une quelconque des revendications 1 à 4, **caractérisé en ce qu'**une extrémité (33) du deuxième élément de traction élastique (31) est, après avoir entouré la partie avant-pied ou mi-pied (23, 24), reliée ou fixée de manière amovible à la partie du deuxième élément de traction (31) s'étendant diagonalement par-dessus le coup de pied (32).

7. Bas de contention pour voûte plantaire selon la revendication 1 ou 2, **caractérisé en ce qu'**une extrémité (28) du premier élément de traction (27) sur la partie arrière-pied (25) et une extrémité (30) du deuxième élément de traction (31) sur la partie arrière-pied (25) peuvent être reliées l'une à l'autre ou bien sont disposées ou peuvent être reliées l'une à l'autre dans la zone talon (26).

8. Bas de contention pour voûte plantaire selon l'une quelconque des revendications précédentes, **caractérisé en ce qu'**au moins un élément de traction élastique (27, 31, 34) est incorporé dans le matériau textile ou est prévu d'être disposé de manière fixe sur une face extérieure ou intérieure du matériau textile.

9. Bas de contention pour voûte plantaire selon l'une quelconque des revendications précédentes, **caractérisé en ce qu'**une extrémité (28, 29, 30, 36) dudit au moins un élément de traction élastique (27, 31, 34) est reliée de manière fixe au matériau textile (22) et que l'autre extrémité (29, 33, 35) est, en tant qu'au moins un élément de traction élastique (27, 31, 34), fixée de manière fixe au matériau textile (22).

10. Bas de contention pour voûte plantaire selon l'une quelconque des revendications précédentes, **caractérisé en ce qu'**au moins une extrémité (28, 29, 30, 33, 35, 36) dudit au moins un élément de traction élastique (34) peut être reliée de manière amovible au matériau textile (22).

11. Bas de contention pour voûte plantaire selon l'une quelconque des revendications précédentes, **caractérisé en ce qu'**au moins une extrémité amovible (28, 29, 30, 33, 35, 36) dudit au moins un élément de traction (27, 31, 34) est, en vue du réglage d'un effort de précontrainte, fixée de manière amovible au matériau textile (22).

12. Bas de contention pour voûte plantaire selon l'une quelconque des revendications précédentes, **caractérisé en ce que** ledit au moins un élément de traction (27, 31, 34) est réalisé sous la forme d'un élément d'accumulation d'énergie, en particulier d'une bande ayant l'élasticité du caoutchouc.
